Europäisches Patentamt

European Patent Office

Office européen des brevets

(19)

(11) Numéro de publication : **0 507 700 A1**

(12) **DEMANDE DE BREVET EUROPEEN**

(21) Numéro de dépôt : **92420093.4**

(22) Date de dépôt : **27.03.92**

(51) Int. Cl.$^5$ : **A61F 15/00, A61F 13/08**

(30) Priorité : **05.04.91 FR 9104535**

(43) Date de publication de la demande :
**07.10.92 Bulletin 92/41**

(84) Etats contractants désignés :
**AT BE CH DE DK ES GB GR IT LI LU MC NL PT SE**

(71) Demandeur : **MOLINIER S.A.**
**rue des Siccards**
**F-42340 Veauche (FR)**

(72) Inventeur : **Delannoy, Robert**
**3 rue Chernoviz**
**F - 75016 Paris (FR)**

(74) Mandataire : **Dupuis, François et al**
**Cabinet Laurent et Charras, 3 Place de**
**l'Hôtel-de-Ville, BP 203**
**F-42005 St. Etienne Cédex 1 (FR)**

(54) **Dossier de prescription d'un bas de contention ou d'une bande de contention.**

(57)    Le dossier comprend :
— au moins une partie (4) apte à recevoir une série (2) de fiches produits (2a) (2b) (2c) (2d), chaque fiche comporte d'une part, des indications sur les bas et les bandes, en fonction du cas pathologique à traiter et de la force de compression exercée par le bas et la bande et, d'autre part, une équivalence des caractéristiques déterminées de la force de compression exercée par le bas et la bande, cette force étant indiquée par un repère commun.
— au moins une partie (1d) apte à recevoir une série de feuilles d'ordonnance (3), comportant notamment les différentes équivalences des caractéristiques des forces de compression exercées par le bas et la bande.

FIG.1

EP 0 507 700 A1

Il est connu d'employer pour le traitement des maladies veineuses notamment, soit des bandes de contention, soit des bas de contention. Généralement, une bonne contention est obtenue en alliant l'efficacité thérapeutique à l'acceptation de son port par le patient. Dans ces conditions, il n'est pas logique de traiter tous les patients et les différents cas pathologiques, avec une seule forme de contention.

Or, il apparaît que la plupart des prescripteurs proposent à leur patient, uniquement des bandes ou uniquement des bas, mais ne leur proposent pas l'une ou l'autre solutions en fonction des critères sus-indiqués. En effet, le prescripteur ne dispose pas de moyens ou d'un outil de travail lui permettant d'une manière simple, sûre et efficace, de proposer, au choix, une bande ou un bas, en fonction de la force de contention que l'on désire.

Cette difficulté est accrue, compte-tenu du fait que les unités de mesure pour déterminer la force de contention d'une bande ou d'un bas, ne sont pas les mêmes. La force de contention d'une bande est généralement, mesurée en cN/cm, tandis que celle d'un bas est généralement, mesurée en hPa ou en mmHg.

On a proposé, pour simplifier la prescription des bas, de codifier la force de contention. Pour chaque plage de valeur, correspondant à une fourchette inférieure et supérieure, d'une certaine force de contention, sont attribués des repères. Par contre, une telle codification n'existe pas pour les bandes.

Or, il apparaît que dans l'un ou l'autre cas, la bande et le bas délivrent une compression résultant notamment de l'élasticité de la bande ou du bas.

Le problème que se propose de résoudre l'invention est d'avoir une codification commune entre les bas et les bandes, pour permettre au prescripteur de sélectionner, en fonction du cas pathologique à traiter et, par conséquent, de la force de compression exercée, indifféremment, un bas ou une bande de contention, tout en respectant les souhaits du patient et en ayant pour objectif d'obtenir une très grande efficacité thérapeutique.

Pour résoudre un tel problème, il a été conçu et mis au point un dossier qui comprend :
– au moins une partie apte à recevoir une série de fiches produits, chaque fiche comporte, d'une part, des indications sur les bas et les bandes, en fonction du cas pathologique à traiter et de la force de compression exercée par le bas et la bande et, d'autre part, une équivalence des caractéristiques déterminées de la force de compression exercée par le bas et la bande, cette force étant indiquée par un repère commun.
– au moins une partie apte à recevoir une série de feuilles d'ordonnance ou de prescirption, comportant notamment les différentes équivalences des caractéristiques des forces de compression excercées par le bas et la bande.

Il en résulte une équivalence de compression entre les bas et les bandes, permettant ainsi d'appliquer avec précision la force de contention choisie. A chaque bande, correspond un bas de la même qualité thérapeutique et inversement.

Un tel problème est résolu en ce que chaque fiche produit comprend un tableau d'équivalence de la même force de contention entre le bas et la bande.

Pour résoudre le problème posé de prescrire un bas ou une bande chaque feuille d'ordonnance comprend deux tableaux récapitulatifs, l'un pour le bas, l'autre pour la bande, chaque tableau représentant une synthèse des tableaux que présente chaque fiche produit.

Compte-tenu du problème posé, d'obtenir une équivalence de compression entre les bas et les bandes, le repère commun est constitué par un chiffre ou une lettre qui correspond, pour le bas, à une unité de pression et, pour la bande, à une unité de force.

Avantageusement, pour résoudre le problème posé de pouvoir sélectionner d'une manière sûre et efficace les caractéristiques d'un bas et d'une bande en fonction de la force de contention désirée et prescrire en conséquence, soit un bas, soit une bande, le dossier se présente sous la forme d'un classeur, la série de fiches produits étant reliées entre elles, d'une manière articulée, tandis que les feuilles d'ordonnance sont disposées, en liasse, dans une pochette rapportée à l'intérieur de l'un des volets du classeur.

Un autre problème que se propose de résoudre l'invention est de pouvoir prescrire la bonne taille du bas ou de la bande, en fonction de la partie du membre considéré.

Un tel problème est résolu en ce que la face interne de l'un des volets du classeur présente un agencement pour recevoir un moyen de mesure.

L'invention est exposée, ci-après plus en détail à l'aide des dessins annexés, dans lesquels :

La figure 1 est une vue en perspective d'une forme de réalisation du dossier.

La figure 2 est une vue de face à caractère purement schématique du recto de l'une des fiches produits, tandis que la figure 3 montre le verso de cette fiche.

La figure 4 est une vue de face à caractère purement schématique montrant un exemple de réalisation d'agencement d'une feuille d'ordonnance.

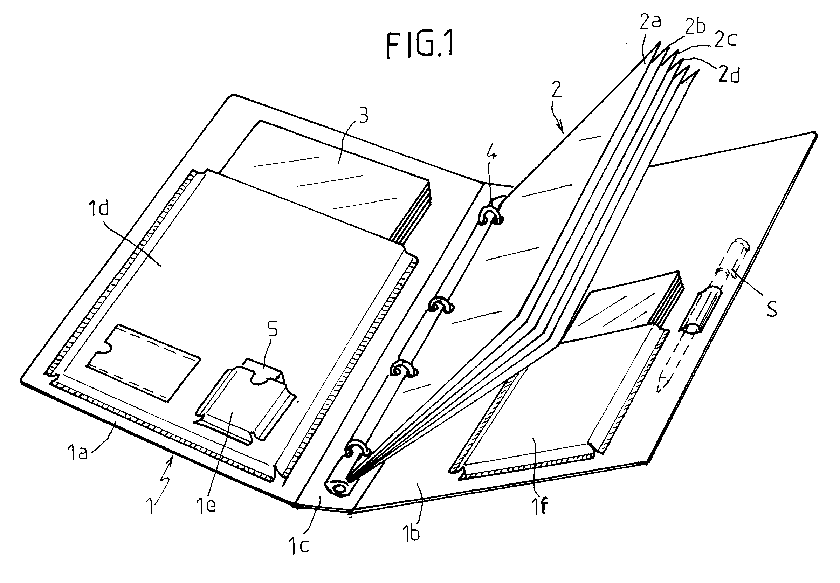

Comme le montre la figure 1, le dossier se présente sous la forme d'un classeur désigné dans son ensemble par (1). Ce classeur peut, par exemple, être du type de ceux comportant deux volets (1a) et (1b) articulés à un volet commun (1c). ce volet (1c) présente des agencements tels que des anneaux (4), sans pour cela exclure d'autres moyens, permettant d'une manière parfaitement connue, la liaison d'une manière séparée, d'un nombre quelconque de feuilles. Notamment, comme il sera indiqué dans la suite de la description, ces anneaux (4) permettent la mise en place d'une série (2) de fiches produits (2a) (2b) (2c) (2d).

Une autre partie du classeur, notamment la face interne du volet (1a), présente une pochette rapportée (1d) apte à recevoir une liasse de feuilles (3) constituant des feuilles d'ordonnance.

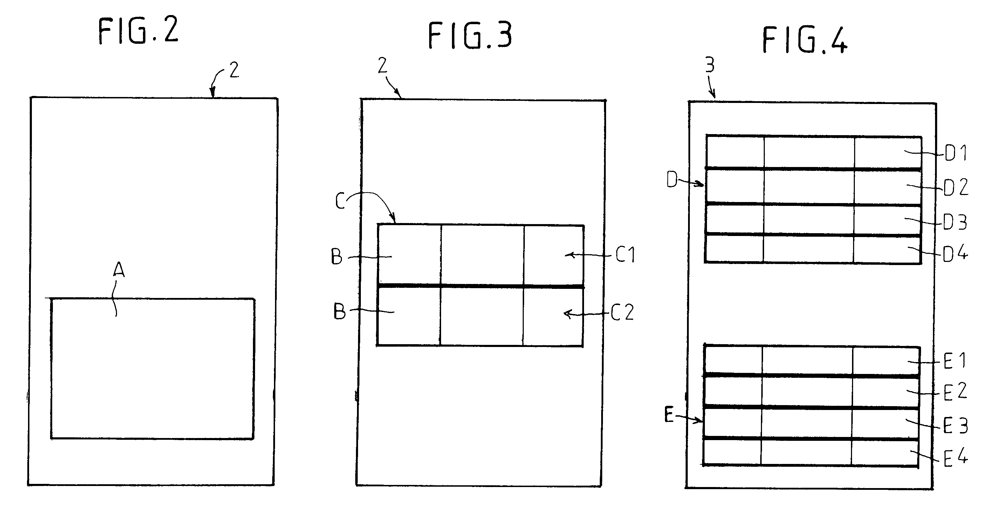

chaque fiche produit comprend des indications thérapeutiques (A) sur les bas et les bandes, en fonction du cas pathologique à traiter et de la force de compression exercée par le bas et la bande. Par exemple, ces indications (A) apparaissent sur le recto de chacune des fiches produits (2a) (2b) (2c) (2d) (figure 2).

En outre, chaque fiche produit comporte une équivalence des caractéristiques déterminées, de la force de compression exercée par le bas et la bande, cette force étant indiquée par un repère commun (B). Dans ce but, chaque fiche produit comprend un tableau d'équivalence (C) de la même force de contention entre le bas et la bande. Ce tableau (C) apparaît par exemple, sur le verso de chacune des fiches (2a) (2b) (2c) (2d) (figure 3).

Comme le montre la figure 3, le tableau (C) comprend essentiellement deux lignes (C1) (C2) correspondant à la même force de contention, pour la bande et le bas. Chacune de ces lignes peut être subdivisée en d'autres lignes et/ou colonnes pour indiquer, par exemple, les caractéristiques, modèles, coloris et autres indications correspondant à la bande et au bas considérés, compte-tenu de la force de contention indiquée. Dans chacune de ces lignes, est indiqué le repère commun (B) correspondant à la force de contention souhaitée.

Par exemple, ce repère commun est constitué par un chiffre ou une lettre qui correspond, pour le bas, à une unité de pression et, pour la bande, à une unité de force.

Avantageusement, les bas et les bandes sont répartis en quatre catégories repérées 1, 2, 3, 4, correspondant chacune à une pression et à une force. On se réfère au tableau ci-dessous, qui montre les équivalences de force de contention, entre un bas et une bande. A chaque force de contention, est affecté le repère commun. Les mesures indiquées dans ce tableau sont données à titre indicatif et ne doivent pas être considérées comme strictement limitatives.

| REPERES COMMUNS | BAS | BANDES |
|---|---|---|
| 1 | de 13 à 20 hPa | de 20 à 45 cNcm |
| 2 | de 20 à 27 hPa | de 46 à 100 cNcm |
| 3 | de 27 à 48 hPa | de 101 à 160 cNcm |
| 4 | > 48 hPa | > 160 cNcm |

Il en résulte, selon cet exemple illustré, que le classeur comprend quatre fiches produits (2a) (2b) (2c) (2d) correspondant chacune à une force de contention 1 ou 2 ou 3 ou 4.

Compte-tenu de l'agencement des fiches produits, chaque feuille d'ordonnance (3) comprend deux tableaux récapitulatifs (D) et (E) correspondant, l'un, aux caractéristiques des bas et, l'autre, aux caractéristiques des bandes. Chaque tableau (D) et (E) constitue une synthèse des tableaux (C), que présente chacune des fiches produits. A cet égard et comme le montre la figure 4, chacun des tableaux est subdivisé en quatre parties (D1) (D2) (D3) (D4) et (E1) (E2) (E3) (E4) correspondant aux repères 1, 2, 3 et 4, indiqués précédemment pour les fiches produits.

Ces différentes feuilles d'ordonnance (3) se présentent en liasse et sont insérées dans la pochette (1d) du classeur.

Ainsi réalisé, ce dossier de prescription, selon les caractéristiques de l'invention, constitue un outil de travail particulièrement efficace et simple d'utilisation. En fonction du cas pathologique à traiter, le praticien regarde les indications thérapeutiques proposées (A) qui apparaissent sur chacune des fiches produits, et consulte le tableau (C) correspondant à la force de contention désirée. Il suffit ensuite, après avoir sélectionné les caractéristiques du bas ou de la bande, de les reporter dans les feuilles d'ordonnance (3).

Compte-tenu de l'application envisagée et dans le but de pouvoir prescrire la bonne taille du bas ou de la bande en fonction du membre à traiter, la face interne de l'un des volets du classeur (1) présente un agencement pour recevoir un moyen de mesure. Par exemple, ce moyen peut être constitué par un mètre (5) logé dans une poche (1e) que présente en surépaisseur la pochette (1d) . De même, l'autre volet (1b) du classeur, peut présenter tout type d'agencement pour recevoir d'autres éléments, tel qu'un stylo (S), ainsi qu'une autre pochette (1f) apte à recevoir des fiches portant des indications diverses.

Les avantages ressortent bien de la description, en particulier on souligne et on rappelle :

– Les équivalences de compression obtenues entre les bas et les bandes permettent d'appliquer avec précision, la force de contention choisie, étant donné qu'à chaque bande correspond un bas et inversement, de la même qualité thérapautique.

– La possibilité de prescrire en fonction de chaque cas pathologique à traiter, indifféremment, un bas ou une bande.

– L'efficacité thérapeutique obtenue.

– La simplicité d'utilisation.

## Revendications

**-1-** Dossier de prescription d'un bas de contention ou d'une bande de contention, comprenant :

– au moins une partie (4) apte à recevoir une série (2) de fiches produits (2a) (2b) (2c) (2d), chaque fiche comporte, d'une part, des indications sur les bas et les bandes, en fonction du cas pathologique à traiter et de la force de compression exercée par le bas et la bande et, d'autre part, une équivalence des caractéristiques déterminées de la force de compression exercé par le bas et la bande, cette force étant indiquée par un repère commun (B).

– au moins une partie (1d) apte à recevoir une série de feuilles d'ordonnance (3), comportant notamment les différentes équivalences des caractéristiques des forces de compression excercées par le bas et la bande.

**-2-** Dossier selon la revendication 1, caractérisé en ce que chaque fiche produit (2a) (2b) ((2c) (2d) comporte un tableau (C) qui comprend essentiellement deux lignes (C1) (C2) correspondant à la même force de contention, pour la bande et le bas, chacune de ces lignes est subdivisée en d'autres lignes et/ou colonnes pour indiquer les caractéristiques correspondant à la bande et au bas considérés, compte-tenu de la force de contention indiquée, dans chacune de ces lignes étant indiqué le repère commun (B) correspondant à la force de contention souhaitée.

**-3-** Dossier selon la revendication 2, caractérisé en ce que chaque feuille d'ordonnance (3) comprend deux tableaux récapitulatifs (D) et (E), correspondant l'un, aux caractéristiques des bas, l'autre, aux caractéristiques des bandes, chacun des tableaux étant subdvisé en plusieurs parties (D1) (D2) (D3) (D4) (E1) (E2) (E3) (E4) (E4) correspondant aux repères communs (B).

**-4-** Dossier selon l'une quelconque des revendications 2 et 3, caractérisé en ce que le repère commun est constitué par un chiffre ou une lettre qui correspond, pour le bas, à une unité de pression et, pour la bande, à une unité de force.

**-5-** Dossier selon la revendication 1, caractérisé en ce qu'il se présente sous la forme d'un classeur (1), la série de fiches produits (2) étant reliées entre elles, d'une manière articulée, tandis que les feuilles d'ordonnance (3) sont disposées en liasse dans une pochette (1f) rapportée à l'intérieur de l'un des volets du classeur (1).

**-6-** Dossier selon la revendication 5, caractérisé en ce que la face interne de l'un des volets du classeur (1) présente un agencement pour recevoir un moyen (5) de mesure.

FIG.1

EP 0 507 700 A1

FIG.2

FIG.3

FIG.4

EP 0 507 700 A1

**European Patent Office**

## EUROPEAN SEARCH REPORT

Application Number

EP  92 42 0093

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-2 349 324 (R. W. GEE,SR.)<br>* figures 3,4,7 *<br><br>--- | 1,2,4,5 | A61F15/00<br>A61F13/08 |
| X | DE-A-2 520 274 (H. B. SCHMITTMANN)<br>* figures *<br><br>----- | 1,5 | |

TECHNICAL FIELDS SEARCHED (Int. Cl.5 )

A61F

The present search report has been drawn up for all claims

| Place of search | Date of completion of the search | Examiner |
|---|---|---|
| THE HAGUE | 06 JULY 1992 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)